# EUROPÄISCHE PATENTANMELDUNG

(11) **EP 0 965 347 A2**
(43) Veröffentlichungstag der Anmeldung: **22.12.1999**
(21) Anmeldenummer: 99111748.2
(22) Anmeldetag: 17.06.1999
(51) Int. Cl.: A61K 35/74

(54) **Verwendung von lebensfähigen anaeroben Bakterien zur Herstellung eines Arzneimittels zur Hemmung des Wachstums von sulfatreduzierenden Bakterien**

(30) Priorität: 17.06.1998 DE 19826928
(71) Anmelder: MED Pharma Service GmbH, 12057 Berlin (DE); Novartis Consumer Health GmbH, 81379 München (DE)
(72) Erfinder: Lauer, Eckhard, 12249 Berlin (DE)
(74) Vertreter: VOSSIUS & PARTNER

(57) **Zusammenfassung**

Beschrieben wird die Verwendung von anaeroben lebensfähigen Bakterien zur Herstellung eines Arzneimittels zur Hemmung des Wachstums von sulfatreduzierenden Bakterien. Weiterhin wird die Verwendung von anaeroben lebensfähigen Bakterien, die das Wachstum und/oder die Sulfatreduktion sulfatreduzierender Bakterien hemmen, zur Herstellung eines Arzneimittels zur Behandlung von IBD oder IBD-bedingten Zuständen beschrieben. Vorzugsweise handelt es sich bei den erfindungsgemäß eingesetzten Bakterien um die Gattung Bififdobacterium, die Klasse Actinobacteria oder um gram-positive Bakterien der intestinalen Mikroflora.

## Beschreibung

Die vorliegende Erfindung betrifft die Verwendung von anaeroben lebensfähigen Bakterien zur Herstellung eines Arzneimittels zur Hemmung des Wachstums von sulfatreduzierenden Bakterien. In einer weiteren erfindungsgemäßen Verwendung werden anaerobe lebensfähige Bakterien, die das Wachstum und/oder die Sulfatreduktion sulfatreduzierender Bakterien hemmen, zur Herstellung eines Arzneimittels zur Behandlung von IBD oder IBD-bedingten Zuständen eingesetzt. Vorzugsweise handelt es sich bei den erfindungsgemäß eingesetzten Bakterien um die Gattung Bififdobacterium, die Klasse Actinobacteria oder um grampositive Bakterien der intestinalen Mikroflora.

Die Mikroflora des Intestinaltrakts von Mensch (und Tier) stellt mit einer Größenordnung von 10¹⁴ Zellen, repräsentiert durch über 400 verschiedene Arten, ein komplexes Ökosystem dar. Seine Funktionen und Wechselwirkungen sind eine Voraussetzung für die Gesundheit des Trägers oder Wirts in normaler Umwelt. Der Wirtsdarm selbst ist ein Organ, das funktionell und histologisch bestens an diese Nischen-Bedingungen angepaßt ist (Luckey, 1972; 1984;Mc Farlane, MacFarlane, 1997). Das mutualistische, durch Evolution geprägte Zusammenspiel von Bakterien und Darm ist von individuell und interindividuell variierenden Parametern, Umweltfaktoren und von flexibel geregelter Physiologie (Wirt und Mikroflora) abhängig. Dabei existiert jedoch nicht ein statisches Gleichgewicht, sondern ein Zustand im Rahmen "nichtlinearer Dymanik" (Goldberger, 1996). Die Beurteilung der jederzeit auftretenden und feststellbaren Variationen der "autochthonen" (= wirtsspezifischer Standort) Besiedlung und deren physiologischen bzw. symbiontischen Auswirkungen ist jedoch schwierig; so wird klassisch und summarisch vereinfacht von *Eubiose* und *Homöostase* gesprochen.

Obwohl L. Pasteur schon 1861 die Buttersäure-Gärung und die Anaerobiose (=Leben ohne Sauerstoff) entdeckt hatte, wurden erst seit etwa 1985 für die physiologischen Beziehungen meßbare Parameter gefunden. Diese werden als mikrobiell assoziierte Charakteristiken (*MAC*) bezeichnet (Rolfe, 1984; Midtvedt et al., 1988; Leijonmarck et al., 1990). Dazu zählen beispielsweise die Besiedlungsresistenz gegen allochthone Bakterien, Abbau von Mucinen, Stimmulation des Immunsystems, trophe Mucosa-Differenzierung, Bildung kurzkettiger Fettsäuren (hauptsächlich Essig-, Propion- und Buttersäure) und aneerobes Redoxpotential.

Der symbiontische Charakter der Mikroflora wird durch das Vorkommen bestimmter Bakterien, z.B. der Bifidobakterien bestimmt, und dadurch unterstrichen, daß verschiedene Arten oder Biotypen von Bifidobakterien bei Tier und Mensch artspezifisch gebunden verbreitet sind. So kommen z. B. *Bifidobacterium bifidum, B. adolescentis, B. breve, B. catenulatum, B. infantis*, und *B.longum* regulär. nur beim Menschen, *B*. *magnum, B*. *pseudolongum, B. thermophilum, B. suis* u. a. dagegen nur beim Tier vor (Mitsuoka, Kaneuchi, 1977; Scardovi, 1986). Die dafür notwendige Selektion beruht anscheinend auf der Fähigkeit der Bakterien, Darm-Mucine als spezifische Andockstellen (Lektine) erkennen und als Substrate nutzen zu können (Bernet et al., 1993; Hoskins et al., 1985; MacFarlane et al., 1997; Salyers et al., 1977; Sato et al., 1982). Außer als vorrangige Essigsäure-Bildner (s. unten) sind Bifidobakterien auch von gewisser nutritiver Bedeutung; sie bilden Vitamine der B-Serie sowie einige Aminosäuren, z. B. Alanin, Valin, Asoaraginsäure und Threonin (Ballongue, 1993).

Der Dickdarm (Kolon) des Menschen, der anaerobe Teil des Darms, stellt den Ort der Hauptaktivität und -masse der Mikroflora dar. Die Kontrolle und Konsequenz der bakteriellen Fermentationsaktivität in diesem Teil wurde kürzlich eingehend dargestellt (Cummings, MacFarlane, 1991; Miller, Wolin, 1996). Es zeigte sich, daß kurzkettige Fettsäuren zu über 90% durch die Wirts-Mucosa resorbiert und extraintestinal seitens des Wirts zur Synthese und Energiegewinnung genutzt werden können. Neben dem symbiontischen Nutzeffekt produziert die Mikroflora allerdings auch Gifte, Toxine oder sonstige "Xenobiotica" (Ito et al., 1992). Um diese zu entgiften, besitzen die Schleimhaut, die Mucosa-Zellen des Kolons, auch Colonozyten (COL) genannt, geeignete, intrazelluläre Enzymsysteme, ähnlich der Leber (Roediger, Babidge, 1997). Hierin und insbesondere in der Abwehr von Infektionen werden COL immunologisch von Mastzellen unterstützt (Crowe et el., 1997), und es besteht eine rege "Immunkonversion" mit der Mikroflora (Pulverer et al., 1997). Dadurch wird das Immunsystem angeregt bzw. stimuliert. Die Mucosa selbst ist, anders als die Leber, ein durch geregelte Zellerneuerung äußerst regeneratives Gewebe, das ein Menge zusätzlicher Energie benötigt (Larson et al., 1990) und daher empfindlich auf Veränderungen reagiert.

Die Verdauung stellt jedoch auch ein Glied der biologischen Nahrungskette bzw. des Mineralisierungskreislaufs der Erde dar. Reste des Nahrungsbreis und abgestoßenes Zellmaterial werden im Kolon teils zu Fettsäuren umgewandelt, teils auch vollständig zu Kohlensäure, Methan, Ammoniak, Wasser, Wasserstoff und Schwefelwasserstoff abgebaut, also "mineralisiert". Für diesen Prozeß sind spezialisierte Bakterien verantwortlich, z. B. Sulfat-reduzierende Bakterien, Methan-Bakterien und acetogene Eubakterien, die auch ubiquitär in natürlichen Gewässern verbreitet vorkommen. Es ist also keine Besonderheit und auch kein Kennzeichen einer "dysbiotischen" Störung des Systems, wenn sich solche Spezialisten auch in der Masse der normalen Mikroflora befinden. Ihre vielfältig bedeutsame Rolle wird erst allmählich bewußt (Zehnder, 1988; Florin, 1991; Grimble, 1989; Gibson et al.; 1990; MacFarlane et al., 1992; Christi et al., 1995; Doré et al., 1995). Es ist bemerkenswert, daß diese Anaerobier, ebenso wie z. B. Bifidobakterien, bereits im Säuglingsalter erworben werden (Hudson, Roberts, 1993).

Es ist bekannt, daß Änderungen qualitativer und quantitativer Art, z.B. durch antibiotische Therapie, zu einer Verschiebung (Dysbiose) der physiologischen Besiedlung führen und akute Störungen der Abwehrfunktionen und Homöostase bewirken. So wird schon seit der Jahrhundertwende über den probiotischen Effekt von Bifidobakterien, insbesondere bei akuten Darmstörungen, spekuliert, und Bifidobakterien werden seit vielen Jahren zur Sanierung der Mikroflora (DE 37 16 938 A1; BE 880 027 und JP 01066124 A) bei Darmerkrankungen (DE 37 16 938 A1; BE 880 027; JP 04273823; EP 0 159 891 und JP 52151787), zur Behandlung von Motilitätsstörungen (Diarrhoe oder Obstipation) (JP 04273823 A und JP 52151787), zur Aktivierung des Immunsystems (JP 05051321 A) oder bei Infektionen mit pathogenen Krankheitserregern wie etwa Clostridium perfringens (BE 880 027 und JE 01121219 A) eingesetzt.

Änderungen qualitativer und quantitativer Art oder dysbiotische Störungen können jedoch nicht als kausale Erklärung jahrzehntelanger entzündlicher Infiltrationen und ulcerierender Veränderungen der Mucosa, irgendwo lokalisiert zwischen Mund und After, mit unterschiedlicher Symptomatik, dienen. Dieser chronisch-pathologisch, atrophierte Organzustand, allgemein als "Inflammatoy Bowel Disease" (IBD) bezeichnet, tritt unabhängig vom Status der mikrobiellen Besiedlung des Intestinaltrakts auf. Das typischerweise auftretende immunologische Ungleichgewicht im Verhältnis von pro-inflammatorischem Interleukin IL-1 zu anti-inflammatorischem IL-1ra und die nachfolgenden inflammatorischen Reaktionskaskaden sowie die stark erhöhte Collagen-Produktion in Muscularis-propria-Zellen und Fibroblasten bei allen Formen der IBD ist nicht mit spezifischen Milieu-Veränderungen zu erklären. Die unspezifischen Organerkrankungen der IBD sind z.B. Morbus Crohn, Colitis ulcerosa, kollagene Colitis, mikroskopische Colitis, sowie extraintestinale Erscheinungen wie juvenile Polyarthritis oder Sacroileitis und Spondylitis am Achsenekelett, Weichteilschwellungen, Osteoporose und Knorpelreduktion an peripheren Gelenken (Rohde, Brackertz, 1984). Die chronischen und i. d. R. schubweise auftretenden Symptome der IBD bzw. die entzündliche Infiltration von Makrophagen in der Lamina propria der Mucosa reagieren nicht auf übliche therapeutische Mittel oder Maßnahmen bei dysbiotisch bedingtem Durchfall oder Malabsorptionssyndrom: Gabe von Antibiotika, Absetzen der Antibiotika-Therapie, Behandlung mit Glucose-Salz-Lösungen oder Wismuth-Verbindungen, Chemotherapie, "Symbiose-Lenkung" oder diätetischen Maßnahmen (Caspary, 1982). Die Ätiologie der IBD ist somit ungeklärt (Miller, Ehms, 1981; Allen et al., 1996), jedoch sprechen Fakten überwiegend für eine mikrobielle Ursache (Chiba et al., 1997; Favier et al., 1997), möglicherweise immunpathologisch verstärkt durch bakterielle Endotoxine (Brandtzaeg et al., 1997; Rietschel, 1997; Jirillo et al., 1995). Wegen der unklaren Ätiologie gibt es bisher weder eine prophylaktische, noch eine kausale Therapie der IBD. Nur symptomatische Behandlung ist bisher möglich, entweder rein anti-inflammatorischer bzw. immunsuppressiver Art mit Salicylazosulfapyridin bzw. 5-Aminosalicylsäure mit Cortikoiden, wie Prednison oder gar mit Weihrauch-Tabletten (Boswelliasäure). Der Erfolg ist meist fraglich und so bleibt schließlich nur noch der operative Eingriff. Die Verwendung von Extrakten oder lebenden Zellen von *E. coli* oder *Lactobacillus* zur Stimulierung der Immunabwehr gilt als obsolet (Meyers, 1996) bzw. als fraglich (Malin et al., 1996), da insbesondere Zellwandbestandteile dieser Bakterien, nämlich Lipopolysaccharid und/oder Murein, als immunpathogenetische Auslöser des *circulus vitiosus* IBD nach dem derzeitigen Stand der wissenschaftlichen Erkenntnisse angesehen werden. Wie jedenfalls auch ersichtlich, wird eine mögliche kausale Rolle der bakteriellen Fermentationsaktivität der intestinalen Mikroflora als Ursache nicht diskutiert.

Die überzeugendste Erklärung für eine mögliche bakterielle Ursache kam von dem australischen Physiologen Roediger (1980a; 1980b; 1982a; 1982b; 1986; 1997). Er zeigte, daß die aus der Kolon-Fermentation stammenden Fettsäuren als primäre Energiesubstrate für das Wachstum und die histologische Integrität der Mucosa notwendig sind. Sie werden Coenzym-A-abhängig (β-Fettsäureoxidation) in den Mitochondrien der COL metabolisiert. Roediger zeigte, daß diese Aktivität bei *Colitis ulcerosa* um 40 % gemindert ist. Roediger und Nance (1986) formulierten die Hypothese, daß Schwefelverbindungen aus dem Darmlumen diese Reaktion blockieren und zu einer chronisch degenerierten Mucosa führen. Florin und Mitarbeiter (1990) wiesen konsequent auf die Entstehung von Schwefelwasserstoff bei der dissimilatorischen Sulfatreduktion durch Aktivität von Sulfat-reduzierenden Bakterien (SRB; zur Phylogenie, s. Fowler et al., 1986) in der Mikroflora hin. Diese Aktivität als Ursache konnte von Mikrobiologen und Physiologen weiter gestützt werden.

SRB, z. B. *Desulfovibrio desulfuricans*, sind in der Mikroflora des Menschen fast normal anzutreffen. Diese hohe Verbreitung ist jedoch offensichtlich nur auf die Industrie- bzw. Hochzivilisationsländer beschränkt (Gibson et al., 1988). Ein großer Teil der SRB gelangt sicherlich über die Gülle-Düngung in die Nahrungskette. Vor allem Säuglinge, die nicht gestillt werden, werden schon frühzeitig mit SRB (und mit Methan-bildenden Bakterien) besiedelt (Baquero et al., 1988; Hudson, Roberts, 1993). Auch SRB in Wasserleitungen aus Eisen-haltigem Material können ein Grund für Ökonomie- bzw. sozial-gebundene Verbreitung der SRB sein (Zehnder, 1988; Zinkevich et al., 1994) Eisenverbindungen selbst spielen eine negative Rolle: Sie fördern nicht nur das Wachstum von SRB durch sogenannte Eisenreduktion (Zehnder, 1988), sondern auch das von *Bacteroides* und Enterobakterien, während Bifidobakterien gehemmt werden (Rowland et al. 1993).

Obwohl SRB meist über Jahrzehnte dem Wirt keine Beschwerden bereiten, stehen sie im Verdacht, eine potentiell pathogene Rolle spielen zu können, wie dies für *Desulfovibrio* unstrittig ist (McDougall et al., 1997). *Desulfovibrio* sind Bakterien, die korkzieherartig geformt sind, die sich sehr gut mithilfe von Geißeln bewegen und sich somit vehement in die Mucosa bohren können, deren Kittleisten aufzubrechen vermögen und - zusammen mit Bakterien der Mikroflora - das Gewebe ulcerieren lassen können. Ähnliche Effekte sind von *Helicobacter pylori* im Schleim des Magens bekannt. Bei normaler Besiedlung und unter physiologisch ausgeglichenen Bedingungen findet ein harter Konkurrenzkampf zwischen SRB, Methanbildnern und sogenannten Acetogenen um Wasserstoff statt (Pochart et al., 1992; Christl et al., 1995). Wasserstoff wird von SRB mit höherer Affinität verwertet, so daß die letzteren beiden Bakteriengruppen gehemmt werden, solange genügend Sulfat zur Verfügung steht (Zehnder, 1988). Nutritiv zugeführtes Sulfat wird im Darm nur langsam resorbiert, so daß es auch das Kolon erreicht (Gomez et al., 1995) und von SRB genutzt wird. Als Kohlenstoff-Quelle wird Laktat (jedoch nicht Acetat, Propionat oder Butyrat) von Desulfovibrio sehr gut zum Wachstum genutzt. So kann bei erhöhten Konzentrationen von Laktat, z. B. bei erhöhter Enterokokken-Besiedlung (van der Wiel-Korstanje, Winkler, 1975), und von Sulfat vermehrt H₂S gebildet werden (Florin, 1991; Willis et al., 1996).

Große Mengen an Schwefelwasserstoff (H₂S) fallen nur bei dissimilatorischer Sulfatreduktion, aber nicht bei proteolytischer Zersetzung von Eiweiß, an. H₂S ist ein schweres, der Blausäure vergleichbares Zellgift, ein stark reaktiver Metabolit, der mit Sauerstoff und zahlreichen Radikalen (z.B. Stickoxidradikal NO) sowie mit Thiol-, Acetaldehyd- und Ketogruppen und nicht zuletzt mit freiem oder polypeptidisch gebundenem Cystein zu reagieren imstande ist. Es bildet schwer lösliche Sulfide mit vielen Metallen, die dadurch nutritiv zu knapp werden können. Auch mit dem Blutfarbstoff Hämoglobin reagiert es zu dem Addukt Sulfhämoglobin mit toxischer Auswirkung. Die Reaktivität von H₂S ist derartig vielgestaltig, daß im Intestinaltrakt bisher nur ein sehr kleiner Teil der Auswirkungen von H₂S erkannt wurde. Zum Beispiel ist nicht geklärt, ob H₂S oder daraus resultierende S'-Radikale bestimmte Antibiotika chemisch verändern und diese somit inaktivieren können. Außerdem wird sicherlich durch H₂S-bedingte Degeneration der Mucosa die systemische Wirkung negativ beeinflußt. Möglicherweise läßt sich damit das Versagen der Antibiotika-Therapie (und die Verschleierung der bakteriellen Ursache) bei IBD eher verstehen. H₂S wird im Wirtskörper durch Methylierung (THF- und Vit.-B12-abhängig), durch Reaktion mit Cystein, durch Mercaptan-Bildung (kurzkettige Fettsäuren, Aldehyde) und über Oxidation zu Sulfat in der Leber entgiftet. Diese Reaktionen können wegen des enormen Mehrverbrauchs an Serin, Cystein und Methionin (Methyl-Stoffwechsel) leicht erschöpfen bzw. an der degenerierten Mucosa nicht mehr kontrolliert ablaufen, so daß H₂S sogar akkumuliert. Durch die starke Infiltration mit Makrophagen am Ort der Entzündung und durch die zusätzliche Belastung mit NO und Wasserstoffperoxid entsteht ein unkontrolliertes Reaktionsgemisch. Dieses hat zur Folge, daß H₂S chemisch zu Thiosulfat oder sogar zu Sulfit reoxidiert wird. Beide Verbindungen werden von SRB erneut zu H₂S reduziert, so daß ein circulus vitiosus auch mit wenig verfügbarem Sulfat entsteht. Nicht zuletzt bedeutet die Reaktion mit Glutathion zu Glutathion-Persulfid eine drämatische Verschlechterung des physiologischen Redoxpotentials mit zahlreichen Nebenwirkungen. Die Senkung der Protein-Sulfhydryl- und DNS-Reparaturkapazität in COL, festgestellt bei IBD-Patienten (Markowitz et al., 1988), ist vermutlich die Basis für mögliche Entartung mit Richtung zu Kolonkrebs.

Die metabolische Flexibilität, die zu IBD führt, ist offensichtlich enorm. SRB können weder Mucine, deren Aufgabe der Schutz der Mucosa ist, direkt als Substrat verwerten, noch Sulfatgruppen von Mucinen oder Sulfatiden abspalten. Dies jedoch besorgt ein Teil der Mikroflora und wird darin sogar symbiontisch vom Wirt gefördert. Mucosa und Mucine werden laufend neu nachgebildet, solange ein physiologisches Gleichgewicht (Eubiose) besteht. Die Sulfatgruppen schützen vor schädlichen Zugriff der Proteasen und Glycosidasen von Bakterien. Auch Gallensäuren werden durch Sulfatisierung entgiftet. Möglicherweise versagen die Sulfat-abhängigen Schutzwirkungen, wenn ein durch Sulfatreduktion bedingter Mangel an Sulfat eine ausreichende Sulfatisierung von Gallensäuren und Mucinen nicht mehr zuläßt; der Schutz sinkt mehr oder weniger rasch ab.

Eine kausale Therapie für diesen circulus vitiosus konnte bisher nicht gefunden werden, und der vorliegenden Erfindung lag somit die Aufgabe zugrunde, eine Verwendung bereitzustellen, die einen wirksamen kausalen Therapieansatz erlaubt.

Die Lösung dieser Aufgabe erfolgte durch die Bereitstellung der in den Patentansprüchen gekennzeichneten Ausführungsformen.

Es wurden Bestandteile der Mikroflora auf inhibitorische Aktivität direkt gegen SRB oder gegen deren H₂S-Aktivität geprüft, um sie gegebenenfalls zur metabolischen Stärkung gegen IBD am Menschen verwenden zu können. Optional sollte auch eine Hemmung von negativ wirkenden Begleitkomponenten, wie *Bacteroides fragilis, E. coli* und proteolytisch H₂S-bildenden Clostridien, berücksichtigt werden.

Überraschenderweise wurden Bestandteile der humanen Mikroflora gefunden, die das Wachstum von SRB bzw. deren H₂S-Produktion effizient hemmen, z. B. Bifidobakterien. Dies ist umso überraschender als Desulfovibrio und Bifidobacterium physiologisch und metabolisch so verschieden sind, daß eine derartige Hemmwirkung kaum zu erwarten war, sondern eher umgekehrt eine Hemmung von Bifidobakterien durch H₂S zu erwarten gewesen wäre.

Die vorliegende Erfindung betrifft daher die Verwendung von anaeroben lebensfähigen Bakterien zur Herstellung eines Arzneimittels zur Hemmung des Wachstums von sulfatreduzierenden Bakterien.

Da bei den durchgeführten Untersuchungen Glucose von *Des. desulfuricans* nicht, sondern nur von Bifidobakterien verwertet wurde, war eine artifizielle Hemmung durch einfache Substrat-Konkurrenz ausgeschlossen. Außerdem bilden Bifidobakterien aus Glucose neben Acetat auch L-Laktat. Auch dieses förderte das Wachstum von *Des*. *desulfuricans* nicht, obwohl es in Reinkultur zum Wachstum genutzt wird. Damit ist der beobachtete Hemmeffekt umso bemerkenswerter. Auch der Zusatz von Pyruvat, das durch *Des. desulfuricans* ohne Sulfat-Zusatz verwertet werden kann, änderte an der beobachteten Hemmwirkung durch Bifidobakterien nichts. Da die getesteten Bifidobakterien und Laktobazillen das Medium während des Wachstums vergleichbar auf einen pH-Wert von 4-5 ansäuern (wegen Laktat- bzw. Acetat-Bildung), jedoch nur erstere einen Hemmeffekt zeigten, kann dieser nicht nur aufgrund einfacher pH-Absenkung im Medium erklärt werden. Die pH-Absenkung wird üblicherweise durch einen pH-Indikator sichtbar gemacht. Da der Ausgangs-pH-Wert bei pH 7,2 liegt, wurde Chinablau als Indikator verwendet, und die Ansäuerung unter pH 6 wird durch Blauverfärbung angezeigt. Es wurde bestätigt gefunden, daß ein pH-Wert von 4-6 auch für *Des. desulfuricans* noch keineswegs zu sauer ist. Dieses Ergebnis könnte mit Blick auf die ungeklärte Pathologie von H₂S bedeuten, daß vor allem geschwächte Aktivität von normalen Anaerobiern der Mikroflora kausal zur Senkung der Aktivitätskontrolle von SRB beiträgt und - bei individueller Disposition - zu Zuständen der IBD führt.

Somit zeigte sich, daß mittels Aktivität von bestimmten anaeroben lebensfähigen Bakterien, beispielsweise Bifidobakterien, die Sulfatreduktion sulfatreduzierender Bakterien gehemmt werden kann, beispielsweise durch Hemmung des Wachstums dieser Bakterien, und somit IBD bzw. damit in Zusammenhang stehende Zustände wirksam bekämpft werden können.

In einer Ausführungsform betrifft die vorliegende Erfindung daher die Verwendung von anaeroben lebensfähigen Bakterien, die das Wachstum sulfatreduzierender Bakterien hemmen, zur Herstellung eines Arzneimittels zur Behandlung von IBD oder IBD-bedingten Zuständen. Die vorliegende Erfindung betrifft weiterhin die Verwendung von anaeroben lebensfähigen Bakterien, die die Sulfatreduktion sulfatreduzierender Bakterien hemmen, zur Herstellung des genannten Arzneimittels.

Erfindungsgemäß kann entweder ein einzelner Stamm verwendet werden oder eine Kombination verschiedener Stämme entweder der gleichen Art oder aus verschiedenen Arten oder Gattungen, gegebenenfalls in Kombination mit einem pharmazeutisch verträglichen Träger.

Der hier verwendete Ausdruck "Arzneimittel" bezieht sich auch auf probiotische Mittel. Diese enthalten in der Regel lebensfähige Mikroorganismen (Bakterien, Hefen), die einen positiven Einfluß auf die Entwicklung, Gesundheit oder Ernährung des Wirts ausüben, und diese können Nahrungsmittel bzw. Nahrungsergänzungsmittel diätetischer oder arzneilicher Art sein (z.B. Joghurt, Butter, Biskuits etc.).

Der hier verwendete Ausdruck "anaerobe lebensfähige Bakterien, die die Sulfatreduktion sulfat-reduzierender Bakterien hemmen" betrifft alle Bakterien der Mikroflora des Menschen, die über eine solche Aktivität verfügen. Dazu zählen neben Bifidobakterien beispielsweise auch die anaeroben Gram-positiven Gattungen Clostridium, Coprococcus, Eubacterium, Peptococcus, Peptostreptococcus, Propionibacterium, Ruminococcus, die Klasse der Actinobacteria, sowie einige Lactobacillus-Arten, die in der humanen Mikroflora verbreitet vorkommen, wie L. acidophilus, L. brevis, L. casei, L. crispatus, L. fermentum, L. gasseri, L. intestinalis, L. jensenii, L. johnsonii, L. paraplantarum, L. pentosus, L. rauteri, L. rhamnosus, L. salivarius. Die Bakterien befinden sich in einem Zustand, der es ihnen gestattet, am Wirkort die vorstehend beschriebene Aktivität zu entfalten, vorzugsweise liegen die Bakterien lyophilisiert oder in lipophiler Suspension vor. Zu den an der dissimilatorischen H₂S-Bildung im Dickdarm fähigen SRB gehören Desulfovibrio desulfuricans, aber auch weitere Arten dieser Gattung sowie die Gattungen Desulfotomaculum, Desulfobacter, Desulfomonas und Desulfobulbus. Für die erfindungsgemäße Verwendung geeignete Bakterien kann der Fachmann leicht anhand des in den nachstehenden Beispielen beschriebenen Tests ermitteln. In der erfindungsgemäßen Verwendung kann auch eine Kombination unterschiedlicher Bakterien mit den vorstehenden Eigenschaften eingesetzt werden.

In einer bevorzugten Ausführungsform werden erfindungsgemäß anaerobe lebensfähige Bakterien der Gattung Bifidobacterium, der Klasse Actinobacteria oder Grampositive Bakterien der intestinalen Mikroflora, z.B. der Gattungen Eubacterium oder Ruminoococcus, oder z.B. auch Stämme der Art Clostridium tyrobutyricum verwendet. Geeignete Isolierungs- und Identifizierungsverfahren sind dem Fachmann geläufig (siehe Bergey's Manual of Systematic Bacteriology, Vol. 2, Sneath et al. (Hrsg.), William & Wilkins London 1986).

In einer stärker bevorzugten Ausführungsform werden bei der erfindungsgemäßen Verwendung Bifidobakterien der folgenden Arten: *B. adolescentis*, *B*. *bifidum*, *B*. *breve; B*. *catenulatum, B. dentium, B. infantis, B. longum* oder B. *pseudocatenulatum* eingesetzt.

In einer alternativen Ausführungsform werden erfindungsgemäß Bifidobakterien, die in Kultur Methionin bilden können, verwendet. Der Methionin-Bildung im Darm kommt bei IBD zur Entgiftung von H₂S besondere Bedeutung zu (Roediger et al., 1996) und ist daher bei der Stammauswahl als vorteilhaft zu berücksichtigen. Dabei dient Methionin zur Entgiftung der H₂S-bedingten Hemmung der mitochondrialen β-Fettsäureoxidation in Colonozyten. Bifidobakterien, die über diese Aktivität verfügen, können anhand der nachstehend beschriebenen Verfahren isoliert bzw. bestimmt werden. Die fraglichen Kulturen werden auf einem Minimalmedium mit Ammoniumsulfat als Stickstoffquelle, aber ohne Aminosäure- oder organischen Stickstoffzusatz, kultiviert und analysiert, analog wie bei Matteuzzi et al. (1978) beschrieben; allerdings ist es vorteilhafter, den dort beschriebenen Zusatz von FeSO₄ und MnSO₄ zum Medium wegzulassen und stattdessen 0,1 % (m/v) fein gepulvertes CaCO₃ hinzuzufügen.

Die erfindungsgemäße Verwendung wird vorzugsweise zur Herstellung eines Arzneimittels zur Behandlung von IBD oder IBD-bedingten Zuständen verwendet. Zu IBD-bedingten Zuständen zählen beispielsweise alle Symptome, Erkrankungen oder Folgeerscheinungen von IBD, Morbus Crohn, Colitis ulcerosa, mikroskopische Kolitis, nekrotisierende Kolitis, Pouchitis, sowie extraintestinale Manifestationen, wie periphere bzw. rheumatoide Arthritis, juvenile Arthritis, hypertrophe Osteoarthropatie, Hauterscheinungen, Erkrankungen der Augen und der Organe, der Haut oder Schleimhäute und allergische Reaktionen. Das Arzneimittel kann außerdem zur Behandlung einer Sulfid- oder H₂S-bedingten IBD im Mund (beispielsweise Parodontitis, Gingivitis) verwendet werden. Das Arzneimittel kann auch zur Stärkung der Remissionophase, vorzugsweise nach operativen Eingriffen und zur Stärkung bei nutritiven Mangelerscheinungen verwendet werden.

Das Arzneimittel wird vorzugsweise oral oder rektal verabreicht, entweder in Steckkapselform, in magensaftresistent überzogenen Kapseln, Tabletten, in Form von Suppositorien auf fetthaltiger oder öliger Grundlage oder als Klysma-Rektalinstillation von Suspensionen. Geeignete Träger und die Formulierung derartiger Arzneimittel sind dem Fachmann bekannt. Zu geeigneten Trägern zählen beispielsweise Phosphat-gepufferte Kochsalzlösungen, Wasser, Emulsionen, beispielsweise Öl/Wasser-Emulsionen, Netzmittel, sterile Lösungen etc. Die geeignete Dosierung wird von dem behandelnden Arzt bestimmt und hähgt von verschiedenen Faktoren ab, beispielsweise von dem Alter, dem Geschlecht, dem Gewicht des Patienten, dem Stadium der Erkrankung, der Art der Verabreichung etc.

Die Verabreichung des Arzneimittels erfolgt vorzugsweise über einen längeren Zeitraum, vorzugsweise auch noch nach Abklingen der Krankheitssymptome. Die Tagesdosis für eine als besonders aussichtsreich einzuschätzende Behandlung in der Remissionsphase liegt bei mindestens 500 Mio lebensfähigen Keimen, eine Überschußtherapie mit 3-maliger Dosierung täglich oder gar mit 100 Milliarden Keimen zu Behandlungsbeginn dürfte hierfür besonders geeignet sein.

Darüber hinaus betrifft die vorliegende Erfindung die vorstehend beschriebenen Arzneimittel, die außerdem einen oder mehrere prebiotische Zusätze enthalten, zur Stärkung von gram-positiven, anaeroben Bakterien der intestinalen Mikroflora oder zur prophylaktischen Stärkung von nicht-gestillten Säuglingen bei genetischer Disposition für IBD oder Wachstumsstörungen.

Der hier verwendete Ausdruck "prebiotische Zusätze" betrifft solche Ingredientien, die zur Stärkung von Gram-positiven anaeroben Bakterien der intestinalen Mikroflora dienen, also einen fördernden Einfluß auf bestimmte Bestandteile der Mikroflora und somit indirekt einen gesundheitlich oder nutritiv positiven Effekt auf den Wirt ausüben. Allgemein bekannt sind solche Anwendungen prebiotisch aktiver Komponenten zur selektiven Förderung der "wirtseigenen" Bifidobakterien bei Säuglingen. Eine derartige selektive, "bifidogene" Wirkung wurde z.B. bei Fructo-Oligosacchariden (Hidaka et al., 1986) gefunden, die aus Artischocken gewonnen werden, oder bei dem Polysaccharid Inulin (Wang, Gibson, 1993), während andere Stoffe, wie Lactulose, Lactitol oder "transgalactosylierte Oligosaccharide" (Rowland, Tanaka, 1993) nicht nur Bifidobakterien, sondern weniger selektiv mehrere verschiedene Bestandteile der Mikroflora fördern. Die Kombination von prebiotisch und probiotisch wirkenden Agentien ist in jedem Fall eine besonders günstige Verwendung (Fuller, Gibson, 1997).

Der hier verwendete Ausdruck "Stärkung von gram-positiven, anaeroben Bakterien der intestinalen Mikroflora" bezeichnet eine selektive Wachstumestimulierung bestimmter Bakterien, wodurch diese aufgrund der erhöhten Teilungsrate einen höheren Anteil und eine höhere Aktivität gegenüber anderen Populationsbestandteilen in der intestinalen Mikroflora erringen. Zu den dadurch bewirkten positiven Auswirkungen (MAC, siehe oben) zählen die Absenkung des physiologischen pH-Werts und Redoxpotentials des Darmlumens, die Absenkung putrefaktiver Metaboliten, die erhöhte Besiedlungsresistenz gegenüber besiedlungsfremden Keimen und Erregern, die Zunahme der bakteriellen Versorgung mit nutritiv und physiologisch bedeutsamen Stoffen, wie Vitaminen, sowie kurzkettige Fettsäuren, vor allen Acetat, Lactat, Propionat und Butyrat, und die histologisch ausdifferenzierte Prägung von Colonozyten.

Wegen dieser symbiontischen Effekte der Mikroflora und da das Stillen des Säuglings einen nachgewiesenen prebiotischen und probiotischen Effekt ausübt, hat die WHO insbesondere in Entwicklungsländern seit vielen Jahren das Stillen probagiert. Aber auch in den Industrieländern ist die Entwicklung der normalen Mikroflora seit Einführung der klinisch geübten Entbindungspraxis und seit Einführung der industrialisierten Säuglingsnahrung bei vorzeitigem Abstillen mit Problemen behaftet. Die Entwicklung der Mikroflora gestaltet sich ungeordnet und ist gekennzeichnet durch die Prävalenz einer viel zu breiten, gesundheitlich nicht günstigen Artenvielfalt. Diese schwächt die Entwicklung einer stabilen Mucosa und belastet vorzeitig das Immunsystem im Reifungszustand. Häufig führt dieser Zustand zu einem wohlbekannten Syndrom, das von chronischer "Überwucherung" des Dünndarms und mit anhaltender Diarrhö vergesellschaftet ist. Dies führt zu schlechtem Gedeihen im Kindesalter. Genetische Disposition zu Abwehr- und Enzymdefekten bereiten ernstzunehmende Zustände, die nicht in geeigneter Weise mit Antibiotika- oder sonstiger Therapie zu behandeln sind, sondern eine Domäne probiotischer Mittel geworden sind.

Zur Behandlung von akuten Darmerkrankungen bei Säuglingen und Kindern wird in DE 37 16 938 und BE 880 027 die Verwendung von Bifidobakterien und Lactobacillus beschrieben.

Schließlich betrifft die vorliegende Erfindung die Verwendung der vorstehend beschriebenen probiotisch wirksamen Bakterien, entweder in Verwendung als einzelner Stamm oder in Kombination verschiedener Stämme, entweder der gleichen Art oder aus verschiedenen Arten oder Gattungen. Außerdem betrifft die Verwendung die Kombination mit einem oder mehreren prebiotischen Zusätzen zur Stärkung von Gram-positiven, anaeroben Bestandteilen der intestinalen Mikroflora oder zur prophylaktischen Stärkung von nicht gestillten Säuglingen bei genetischer Disposition für IBD oder Wachstumsstörungen aufgrund einer chronischen Syndrom-Situation.

Die nachstehenden Beispiele erläutern die Erfindung.

### Beispiel 1

### Allgemeine Verfahren

(a) Testkulturen:
   *Des. desulfuricans* DSM 642 stammt von der Deutschen Sammlung von Mikroorganismen und Zellkulturen GmbH, Braunschweig. Die Bifidobakterien, *B. longum* DSM 11913 und *B. spec.* DSM 11914 waren Eigenisolate und wurden am 12. Januar 1998 bei der DSM, Braunschweig, hinterlegt.
(b) Kulturbedingungen und Identifizierung:
   Alle Stämme wurden auf geeignetem Medium angezüchtet. Für Des. *desulfuricans* wurde DSM-Medium 63 (Catalogue of Strains, 5. Ausg., 1993, Deutsche Sammlung von Mikroorganismen und Zellkulturen GmbH, Braunschweig) und Blutagar (nach *Hackenthal-Bierkowski*) folgender Zusammensetzung verwendet (% m/v): Blutagar-Grundsubstrat Nr.2 (Oxoid CM 271), 4,0; Glucose (Merck 8346), 1,0; Chinablau (Merck), <0,03; Defibriniertes Schafsblut (Oxoid FSR 1055), ca. 7,0; pH-Wert: ca. 7,2. Gutes Wachstum erfolgte bei 37°C unter anaeroben Bedingungen innerhalb von 4-6 Tagen. Zur Anaerobiose dienten Anaerobensysteme Oxoid BR 38 und - zum Vergleich - Anaerocult A (Merck). Der Zusatz von je 0,002 g Ascorbat und Thioglycolat pro Blutagar-Platte (ca. 20 ml Medium) verbesserte ebenfalls das Wachstum bei. Erstbeimpfung von *Des. desulfuricans*, ist jedoch bei Subkultur nicht unbedingt erforderlich. Ein Pyruvatzusatz (0,03 g/Platte) im Medium verbesserte ebenfalls das Wachstum von *Des. desulfuricans*. Anhand der H₂S-Bildung konnte festgestellt werden, daß das Medium ausreichend Sulfat enthält. Die Entwicklung von H₂S bildete im Anaerocult-System einen deutlichen, schwarzen Belag am Katalysator (Eisensulfid). Die Bifidobakterien wurden auf DSM-Medium 58 (*Catalogue of Strains*, 5. Ausg., 1993. Deutsche Sammlung von Mikreorganismen und Zellkulturen GmbH, Braunschweig) unter den gleichen anaeroben Bedingungen (Oxoid BR 38 und Merck Anaerocult A) bei 37°C für 2 Tage kultiviert. Zur Stabilisierung der Aktivität wurde dem Medium 0,2 % CaCO₃-Pulver zugesetzt.
   Die Laktobazillen wurden auf DSM-Medium 11 (Catalogue of strains, 5. Ausg., 1993, Deutsche Sammlung von Mikroorganismen und Zellkulturen GmbH, Braunschweig) gezüchtet. Das Wachstum unter anaeroben Bedingungen (unter den gleichen Bedingungen wie oben) bei 37°C erfolgte optimal innerhalb von 2 Tagen.
   Die Identität wurde keim- und koloniemorphologisch und durch biochemische Kriterien abgesichert: H₂S-Bildung (*Des. desulfuricans*); Prüfung des Spektrums vergärbarer Kohlenhydrate (Laktob. und Bifidob.).
(c) Cokultur-Testung auf Blutagar:
   Es wurden zwei allgemein bekannte in-vitro-Tests zur Feststellung einer Hemmwirkung angewandt:
   Frisch kultivierte Desulfovibrio-Kultur auf Blutagar wurde in physiologischer Kochsalzlösung zu etwa 10⁷ KBE/ml suspendiert und davon 0,1 ml mit dem Glas-Spatel auf der Medium-Oberfläche (Blutagar) verteilt. In der Mitte des Nährbodens wurde ein Kolonie-Spot des zu prüfenden Bitidobakteriumstammes mit 5 mm Durchmesser mittels Impföse aufgetragen und anaerob (s. oben) bei 37°C für 4 Tage bebrütet, danach das Wachstum bzw. der Hemmhof beurteilt (Ösen-Spot-Test). Alternativ wurde das Verhalten im "Gegenstrichtest-Verfahren (Kreuzstrichtest)" geprüft. Frisch gewachsene Kolonien von *Des. desulfuricans* wurden mit der Impföse diagonal über die Medium-Oberfläche ausgestrichen. Anschließend wurden frisch gewachsene Kolonien, jeweils von Bitidobakterien- oder Laktobazillen-Teststämmen, rechtwinklig gegen den ersten Ausstrich aufgetragen und anaerob (s. oben) bei 37°C für 4 Tage bebrütet, danach das Wachstum bzw. der Hemmhof ausgewertet.
   Zur Kontrolle des ungehemmten Wachstums dienten Reinkulturen im Parallelansatz. Außerdem wurde in einigen Fällen das Verhalten der um 2 Tage bei Raumtemperatur verlängerten Bebrütung getestet. Es ergaben sich keine wesentlichen Unterschiede zu der 4-tägigen Bebrütung

### Beispiel 2

### Hemmung des Wachstums von Des. desulfuricans durch Bifidobakterien

Die Kultivierung in Reinkultur auf Blutagar (nach Hackenthal-Bierkowski) erwies sich als problemlos. Alle Teststämme, mit Ausnahme des Stammes B. spec. DSM 11914 ließen sich auf dem Medium auch weiterkultivieren. Die Testung im Cokulturverfahren auf diesem Medium verlief ebenfalls problemlos.

Mit beiden Teststämmen der Bifidobakterien konnte eine profunde Hemmung von *Des. desulfuricans,* mit Laktobazillen dagegen keine Hemmung beobachtet werden. Die Hemmhöfe wiesen folgende Durchmesser (mm) auf (siehe Tabelle):

| Test | Teststamm/Des. desulf. | Hemmung (mm) |
|---|---|---|
| Ösen-Spot-Test | B. longum DSM 11913 | 27 |
| | B. spec. DSM 11914 | 20 |
| Kreuzstrichtest | B. longum DSM 11913 | 27 |
| | B. spec. DSM 11914 | 16 |

In weiteren Experimenten wurde festgestellt, daß die Hemmwirkung auch abhängig ist von der Vitalität der Bifidobakterien. Diese erwiesen sich im Vergleich mit *Des. desulfuricans* hinsichtlich der Anforderung an anaerobe Bedingungen und gegenüber dem Kontakt mit Luftsauerstoff als deutlich anspruchsvoller. Dies bedeutet, daß eine Verringerung der Anaerobiose den Hemmeffekt von Bifidobakterien ebenfalls verringert.

### LITERATURLISTE

- Allen, R.N. et al.: Inflammatory Bowel Disease (monograph, 3rd ed.). Churchill Livingstone, Edinburgh (GB) 1996.
- Ballongue, J.: Bifidobacteria and probiotic action, - in: Lactic Acid Bacteria, Ch.13, S. Salminen, A. von Wright (Hrsg.), Marcel Dekker, New York, 1993.
- Baquero, F., et al.: Microbial Ecol. Health Dis. 1 (1988) 101-108.
- Bernet, M.F., et al.: Appl.Environ.Microbiol. 59 (1993) 4121-4128.
- Brandtzaeg, P., et al.: Springer Semin.Immunopathol. 18 (1997) 555-589.
- Caspary, W.F.: Dt.Apothekerzeitung 122 (1982) 45-52.
- Chiba, M., et al.: Gut 41, Suppl. 3 (1997) A12.
- Christl, S.U., et al.: Z.Gastroenterol. 33 (1995) 408-413.
- Crowe, S.E., et al.: Gut 41 (1997) 785-792.
- Cummings, J.H., Macfarlane, G.T.: J.appl.Bacteriol. 70 (1991) 443-459.
- Doré J., et al.: FEMS Microbiol.Ecol. 17 (1995) 279-284.
- Favier, C., et al.: Dig.Dis.Sci. 42 (1997) 817-822.
- Florin, T.H.J.: Clin.Chim.Acta 196 (1991) 127-134.
- Florin, T.H.J., et al.: Gastroenterol. 98 (1990) 170.
- Fowler, V.J., et al.: System.appl.Microbiol. 8 (1986) 32-41.
- Fuller, R., Gibson, G.R.: Scand.J.Gastroenterol. 32, Suppl. 222 (1997) 28-31.
- Gibson, G.R., et al.: J.appl.Bacteriol. 65 (1988) 103-111.
- Gibson, G.R., et al.: Gut 31 (1990) 679-683.
- Goldberger, A.L.: Lancet 347 (1996) 1312-1314.
- Gomez, G.G., et al.: J.Nutr. 125 (1995) 2325-2332.
- Grimble, G.: Gut 30 (1989) 6-13.
- Hidaka, H., et al.: Bifidobacteria Microflora 5 (1986) 37-50.
- Hoskins, L.C., et al.: J.clin.Invest. 75 (1985) 944-953.
- Hudson, M.J., Roberts, A.K.: Microb.Ecol.Health Dis. 6 (1993) 301-308.
- Ito, Y., et al.: Microb.Ecol.Health Dis. 5 (1992) 1-13.
- Jirillo, E., et al.: Microecol.Ther. 25 (1995) 37-41.
- Larson, G., et al.: Microb.Ecol.Health Dis. 3 (1990) 305-319.
- Leijonmarck, C.-E., et al.: Scand.J.Gastroenterol. 25 (1990) 585-593.
- Luckey, T.D.: Am.J.clin.Nutr. 25 (1972) 1292-1294.
- Luckey, T.D.: Microecol.Ther. 14 (1984) 243-249.
- MacFarlane, G.T., MacFarlane, S.: Scand.J.Gastroenterol. 32, Suppl. 222 (1997) 3-9.
- MacFarlane, G.T., et al.: J.appl.Bacteriol. 72 (1992) 57-64.
- MacFarlane, S., et al.: Adv.Dent.Res. 11 (1997) 59-68.
- Malin, M., et al.: Ann.Nutr.Metabol. 40 (1996) 137-145.
- Markowitz, M.M., et al.: Gut 29 (1988) 1680-1686.
- Matteuzzi, D., et al.: Ann.Microbiol.(Inst. Pasteur) 129 B (1978) 175-181.
- McDougall, R., et al.: J.clin.Microbiol. 35 (1997) 1805-1808.
- Meyers, S.: In: Crohn's Disease (monograph), Ch. 31. C. Prantera, B.I. Korelitz (Hrsg.). Marcel Dekker New York, 1996.
- Midrvedt, A.-C., et al.: J.Ped.Gastroenterol.Nutr. 7 (1988) 559-567.
- Miller, B., Ehms, H.: Internist 22 (1981) 379-384.
- Miller, T.L., Wolin, M.J.: Appl.environ.Microbiol. 62 (1996) 1589-1592.
- Mitsuoka, T., Kaneuchi, C.: Am.J.clin.Nutr. 30 (1977) 1799-1810.
- Pochart, P., et al.: FEMS Microbiol.Lett. 98 (1992) 225-228.
- Pulverer, G., et al.: Scand.J.Gastroenterol. 32, Suppl. 222 (1997) 107-111.
- Rietschel, E.Th.: Yakult Symp.Bonn (1997) Abstract.
- Roediger, W.E.W: Lancet Vol.II (1980a) 712-715.
- Roediger, W.E.W: Gut 21 (1980b) 793-798.
- Roediger, W.E.W: Colon and Nutrition, Ch. 2. Falk Symp. H. Kasper, H. Goebell (Hrsg.). MTP Press Lim. Falcon House, Lancaster (GB), 1982a.
- Roediger, W.E.W: Gastroenterol. 83 (1982b) 424-429.
- Roediger, W.E.W: Lancet Vol.I (1986) 1082-1084.
- Roediger, W.E.W., Babidge, W.: Gut 41 (1997) 731-734.
- Roediger, W.E.W., Nance, S.: Br.J.exp.Path. 67 (1986) 773-782.
- Roediger, W.E.W., et al.: Gut 39 (1996) 77-81.
- Roediger, W.E.W., et al.: Dig.Dis.Sci. 42 (1997) 1571-1579.
- Rohde, H.-J., Brackertz, D.: Verdauungskrankheiten 2 (1984) 81-94.
- Rolfe, R.D.: Rev.Infect.Dis. 6, Suppl. 1 (1984) S73-S79.
- Rowland, I., et al.: Microb.Ecol.Health Dis. 6 (1993)
- Rowland, I.R., Tanaka, R.: J.appl.Bacteriol. 74 (1993) 667-674.
- Salyers, A.A., et al.: Appl.environ.Microbiol. 34 (1977) 529-533.
- Sato,J., et al.: Bifidobacteria Microflora 1 (1982) 51-54.
- Scardovi, V.: Genus Bifidobactererium Orla-Jensen 1924, p. 1418-1434 in Bergey's Manual of Systematic Bacteriology, Vol.2, Sect. 15: Irregular, nonsporing Gram-positive Rods. Sneath et al. (Hrsg.), Williams & Wilkins, London, 1986.
- van der Wiel-Korstanje, J.A.A.,: J.med.Microbiol. 8 (1975) 491-501.
- Winkler, K.C.:
- Wang, X., Gibson, G.R.: J.appl.Bacteriol. 75 (1993) 373-380.
- Willis, C.L., et al.: Anaerobe 2 (1996) 117-122.
- Zehnder, A.J.B.: Biology of Anaerobic Microorganisms (monograph). John Wiley & Sons, Toronto (Can.) 1988.
- Zinkevich, V. et al.: IUMS, 17th Int. Congress Prag (1994) Abstract book, p.252

## Patentansprüche

1. Verwendung von anaeroben lebensfähigen Bakterien zur Herstellung eines Arzneimittels zur Hemmung des Wachstums von sulfatreduzierenden Bakterien.

2. Verwendung von anaeroben lebensfähigen Bakterien, die das Wachstum sulfatreduzierender Bakterien hemmen, zur Herstellung eines Arzneimittels zur Behandlung von IBD ("Inflammatory Bowel Disease") oder IBD-bedingten Zuständen.

3. Verwendung von anaeroben lebenafähigen Bakterien, die die Sulfatreduktion sulfatreduzierender Bakterien hemmen, zur Herstellung eines Arzneimittels zur Behandlung von IBD ("Inflammatory Bowel Disease") oder IBD-bedingten Zuständen.

4. Verwendung nach einem der Ansprüche 1 bis 3, wobei die anaeroben lebensfähigen Bakterien Bakterien der Gattung Bifidobacterium, der Klasse Actinobacteria oder gram-positive Bakterien der intestinalen Mikroflora sind.

5. Verwendung nach Anspruch 4, wobei die Gattung Bifidobakterium die Arten *B*. *adolescentis, B. bifidum, B. breve, B. catenulatum, B. dentium, B. infantis, B. longum* oder *B*. *pseudocatenulatum* umfaßt.

6. Verwendung nach Anspruch 4 oder 5, wobei es sich um ein Bifidobakterium handelt, das Methionin bilden kann.

7. Die Verwendung nach einem der Ansprüche 1 bis 6, wobei die anaeroben lebensfähigen Bakterien in Kombination mit einem oder mehreren prebiotischen Zusätzen zur Stärkung der gram-positiven, anaeroben Bakterien der intestinalen Mikroflora verwendet werden.
